# EUROPEAN PATENT SPECIFICATION

(11) **EP 2 288 360 B1**
(45) Date of publication and mention of the grant of the patent: **14.12.2011**
(21) Application number: 09745475.5
(22) Date of filing: 12.05.2009
(51) Int. Cl.: A61K 35/74, C12R 1/25, A23C 9/123

(54) **ISOLATED LACTOBACILLUS PLANTARUM STRAIN INDUCIA DSM 21379 AS PROBIOTIC THAT ENHANCES NATURAL IMMUNITY AND FOOD PRODUCTS AND MEDICINAL PREPARATIONS COMPRISING IT**
ISOLIERTES LACTOBACILLUS-PLANTARUM-STAMM-INDUCIA DSM 21379 ALS PROBIOTIKUM ZUR VERBESSERUNG DER NATÜRLICHEN IMMUNITÄT UND DIESES UMFASSENDE NAHRUNGSPRODUKTE UND MEDIZINISCHE ZUBEREITUNGEN
LA SOUCHE ISOLÉE LACTOBACILLUS PLANTARUM, INDUCIA DSM 21379 COMME PROBIOTIQUE QUI AMÉLIORE LE POTENTIEL DE DÉFENSE NATURELLE DE L'ORGANISME, ET PRODUITS ALIMENTAIRES ET PRÉPARATIONS MÉDICINALES LA COMPRENANT

(30) Priority: 13.05.2008 EE 200800027
(43) Date of publication of application: 02.03.2011
(73) Proprietor: Oü Tervisliku Piima Biotehnoloogiate Arenduskeskus, 51014 Tartu (EE)
(72) Inventor: MIKELSAAR, Marika, EE51005 Tartu (EE); SONGISEPP, Epp, EE50101 Tartu (EE); SMIDT, Imbi, EE61622 Tartumaa (EE); STSEPETOVA, Jelena, EE50707 Tartu (EE); HÜTT, Pirje, EE50110 Tartu (EE); ZILMER, Mihkel, EE50406 Tartu (EE); TRUUSALU, Kai, EE50107 Tartu (EE); KILK, Kalle, EE50404 Tartu (EE)
(74) Representative: Kahu, Sirje
(86) International application number: PCT/EE2009/000006
(87) International publication number: WO 2009/138092

(56) References cited:
- WO-A-03/071883
- SAARELA M ET AL: "Probiotic bacteria: safety, functional and technological properties" BRAUWELT, vol. 84, no. 3, 28 December 2000 (2000-12-28), pages 197-215, XP004314235 ISSN: 0724-696X
- DE VRIES M C ET AL: "Lactobacillus plantarum-survival, functional and potential probiotic properties in the human intestinal tract" INTERNATIONAL DAIRY JOURNAL, vol. 16, no. 9, 1 September 2006 (2006-09-01), pages 1018-1028, XP024963305 ISSN: 0958-6946 [retrieved on 2006-09-01]
- STANTON C ET AL: "Fermented functional foods based on probiotics and their biogenic metabolites" CURRENT OPINION IN BIOTECHNOLOGY, vol. 16, no. 2, 1 April 2005 (2005-04-01), pages 198-203, XP004849212 ISSN: 0958-1669

## Description

### TECHNICAL FIELD

The present invention relates to the field of biotechnology and application in food industry and medicine. The present invention relates to a microorganism *Lactobacillus plantarum* strain Inducia DSM 21379 as probiotic, which produces polyamines, nitric mono-oxide and antioxidative compounds, improves the intestinal barrier function, intestinal mucosa and blood cellular immunity and induces pro-inflammatory cytokines. The invention relates to its use in the composition of a functional food, e.g. in milk products and in a pharmaceutical composition enhancing natural immunity of organism through improvement of the intestinal barrier function and enhancement of intestinal mucosa and blood cellular immunity.

### BACKGROUND ART

During the past decades lactobacilli have widely been used as probiotics in functional foods. Food can be regarded as functional, if beyond adequate nutritional components it contains some natural additives (pre- or probiotics), which beneficially affect one or more target functions in the body, either improving the state of health and well-being and/or reducing disease risk. Probiotics are live microorganisms, which, when administered in adequate scientifically proven amounts, confer a health benefit on the host. Commonly probiotics are used for creating functional foods. Probiotic products may be conventional foods (yoghurt, quark, curds) or dietary supplements (freeze-dried microbial cultures). Probiotics are often used for enhancement of organisms' defence capability.

Enhancement of organisms' natural immunity has become essential in connection with the ageing of population and diseases connected with immunodeficiency (HIV infection, tissue transplantation induced immunosuppression). Infections, disturbances in metabolism and tumorigenic processes are observed more frequently in elderly (Hebuterne X., Gut changes attributed to ageing: effects on intestinal microflora. Curr. Opin. Clin. Nutr Metab Care, 2003; 6 (1):49-54; Wenzel, R.P., Health care - associated infections. Major issues in early years of 21st century. Clin Inf. Dis., 2007; 45: S85-S88). The cause of all of mentioned diseases is the decrease of the capability of several physiological functions of the organism (Timiras, P.S. Physiology of aging: standards for age-related functional competence In: Comprehensive Human Physiology. Greger, R (edt)/ Windhorst, U (eds) Springer Verlag, 1996; pp 2391-2405). Ageing is regarded as a decrease of stress-resistance and weakened immune response. First of all, this affects the counts of defence cells (leucocytes, macrophages, lymphocytes, trombocytes) circulating in human body and the decrease of the activity of phagocytes as well as the decrease of the barrier function of mucosa (Hebuterne X., Gut changes attributed to ageing: effects on intestinal microflora. Curr. Opin. Clin. Nutr Metab Care, 2003; 6 (1):49-54; Fang H., Intestinal microbiota of the elderly. In: Gastrointestinal Microbiology: The Intestinal Microbiota of Elderly Edited by A. Ouwehand and E. Vaughan 2006; pp 75-91).

The permeability of the intestinal mucosa frequently increases on the background of chronic inflammation. The mentioned condition is a basis for the generalization of intestinal infections due to autoallergic reactions and microbial translocation. Microbial metabolites play essential role in the integrity of mucosa, e.g. short chain fatty acids (SCFA), produced by lactic acid bacteria in the colon in the case of fiber-rich (substances of plant origin) diet (Roy CC, Kien CL, Bouthillier L., Levy E. Short chain fatty acids: ready for prime time? Nutr.Clin. Pract., 2006; 21:351-366). Butyrate, produced by eubacteria and clostridia is essential for enhancement of barrier function of the mucosa, likewise the effect of fiber-rich diet on colonic microflora, which has been described in several studies (O'Keefe SJD. Nutrition and colonic health: the critical role of the microbiota. There is strong evidence for the ability of diet to influence the colonic microbiota and cancer risk as measured by epithelial proliferation rate. Current Opin Gastoenterol. 2008; 24: 51-58).

For enhancement of mucosal barrier besides short chain fatty acids also polyamines are essential. Polyamines are linear aliphatic compound, in which amino acids are situated along the structure. Putrescine, spermidine and spermine belong to polyamines (Larqué, M., Sabater-Molina, S. Zamora E. Biological significance of dietary polyamines. Nutrition 2007; 23(1): 87-95). Polyamines are produced by decarboxylation from amino acids ornithine and arginine. Putrescine is produced straight from ornithine; arginine is primarily converted into agmantine which is then converted into putrescine (Halaris A, Plietz, Agmatine: metabolic pathway and spectrum of activity in brain. CNS Drugs, 2007; 21: 885-90). Diamine oxidase metabolizes putrescine into gamma-butyric acid, which is an essential molecule in neurons metabolism.

Besides, arginine serves as the source of nitricmono-oxide (NO); the system NOS-NO could possess hyperemia-dependent protection mechanism against stress-induced damage of intestinal mucosa (Brzozowski T., Brzozowski, Konturek P., C., Sliwowski Z., Drozdowicz D., Burnat G., Pajdo R., Pawlik M., Bielanski W., Kato I., Kuwahara A., Konturek S. J. and Pawlik W.W. Gastroprotective action of orexin-A against stress-induced gastric damage is mediated by endogenous prostaglandins, sensory afferent neuropeptides and nitric oxide. Regul Pept 2008 in press).

The physiological impact of polyamines is targeted to cell growth and differentiation, regulation of immune cells and inflammatory response, polyamines possess also anti-allergic, protein synthesis-stimulating, nucleic acid structure stabilizing and enzyme activity controlling effect Polyamines reduce the development of ulcers in intestine and improve the intestinal mucosa, thus reducing its permeability for macromolecules. Polyamines possess the ability to induce apoptosis, avoiding the hyperproliferation of epithelium and the development of primary cancer cells (Moinard C, Cynober L, De Bandt JP Polyamines: metabolism and implications in human disease. Clin Nutr., 2005; 24: 184-197). Polyamines are produced either endogenously or they are obtained actively from food. Only 20% of consumed putrescine is absorbed from intestine into bloodstream, accompanied by the raise of acetylated putrescine, while majority is metabolized by liver and enterocytes (Milovic V, Odera G, Murphy GM, Dowling RH Jejunal putrescine absorption and the pharmakokinetic/biotransformation of ingested putrescine in humans. Gut, 1997; 41: A63). It is important especially for elderly people, whose endogenous polyamine-synthesizing ability is lowered, to gain polyamines with food. Abundance of putrescine, spermidine and spermine can be found in acetylated or oxidized form in urine (Seiler N Catabolism of polyamines. Amino Acids 2004; 26: 217-233). Putrescine is considered toxic in concentrations of 2000 mg/kg; however, putrescine and cadavereine can also intensify the toxic effect of tyramine and histamine. Therefore, > 300 mg/kg per food is considered the total toxicity of polyamines (Larqué, M., Sabater-Molina, S. Zamora E. Biological significance of dietary polyamines. Nutrition 2007; 23(1): 87-95).

In the case of the damage of epithelial cells, the production of polyamines by the intestinal microflora is considered one of the compensatory mechanisms for modification of immune response and apoptosis regulation. Putrescine is produced by several anaerobes, *Escherichia coli* and lactobacilli. Lactobacilli comprise majority of microflora of the proximal colon (Marteau, P., Pochart, P., Dore, J., Bera-Maillet, C., Bernalier, A. and Corthier, G. Comparative study of bacterial groups within the cecal and fecal microbiota. Appl. Env. Microbiol. 2001; 67, 4939-4942). Lactobacilli produce polyamines through decarboxylation of amino acids, particularly at the high pH of the intestinal content (Lonvaud-Funel A, Biogenic amines in wine: role of lactic acid bacteria. FEMS Microbiol. Letters, 2001, 199: 9-13). On the other hand, strains of *Lactobacillus acidophilus* utilize putrescine and reduce odour of faces (WO 2008/019887, BASF AG).

Japanese patent application JP2006191808 (Toyota Motor Corporation) discloses putrescine production technology with enterobacteria. Probi AB Estonian patent EE03597 discloses pharmaceutical composition that contains *L. plantarum* strains 299 and 299v together with arginine for prevention translocation of intestinal microbes during liver injury. These strains of *L. plantarum* resemble over 70% (REA) to *L. plantarum* strain 299 and they are able to colonise human intestinal mucosa *in vivo.* In this patent no information is available concerning the end products of arginine utilization (putrescine, cadaverine, tyramine, enhancement of NO or antioxidativity) by *L. plantarum,* which are responsible for aforementioned effect. In close relationship with previous patent another Probi AB patent EE04097 discloses the strains *L. plantarum* 299v and 299, which can colonize the mucosa due to mannose-specific adhesins. This patent also does not disclose the polyamines or NO production ability of mentioned *L. plantarum* strains.

However, due to administration of high amounts of arginine daily (3.4 g/daily), significant raise of NO is observed in comparison with lower doses (3.2 g/ daily) (Jablakad A. Checinski P., Krauss H., Mickerbe M. Astcef J. The influence of two different doses of L-arginine oral supplementation on nitric oxide (N=9) concentration and total antioxidant status (TAS) in atherosclerotic patients. Clinical Research, 2004). It has been demonstrated, that *Lactobacillus rhamnosus* GG could enhance NO production in the epithelial cells of the intestine or by proinflammatory cytokines and it has been indicated, that some beneficial effects of *Lactobacillus rhamnosus* GG could be due to the production of NO by macrophages and epithelial cells (Korhonen K, Reijonen TM, Remes K, Malmström K, Klaukka T, Korppi M. Reasons for and costs of hospitalization for paediatric asthma: A prospective 1-year follow-up in a population-based setting. Pediatr Allergy Immunol 2001; 12:331-338). It has been demonstrated, that NO protects mucosa for damages and excessive permeability, arising after reperfusion (Payne D, Kubes P. Nitric oxide donors reduce the rise in reperfusion-induced intestinal mucosal permeability. Am J Physiol. 1993; 265 (1 Pt 1):G189-G195).

USA patent application US20060078595 (Friesland Brands B.V.) discloses method to avoid the excessive permeability of the intestinal barrier in newborns by glutamate and its precursors as well as by polyamines spermidine, spermine, putrescine in the case of different syndromes (malnutrition, allergy, sepsis, translocation of microbes, endotoxemia, viral diarrhoea). *Lactobacillus* Reuteri (BIOGAIA) served as glutamate source.

Only a few probiotics are available for elderly population. The counts of intestinal lactobacilli were higher in the regularly probiotics consuming elderly in comparison with these who did not consume probiotics (Soovares, P., Salusaar L, Kolk H, Sepp E, Kullisaar T, Vihalemm T, Zilmer M, Mikelsaar M. Kas soole laktobatsillide hulk ja söömisharjumused mõjutavad ateroskleroosi riski eakatel isikutel? Eesti Arst, 2007; 9: 704). Probiotics for elderly are usually targeted for the reduction of chronic inflammations by using immune suppressing and IL-10 inducing bacteria (bifidobacteria, *Lactobacillus acidophilus*) e.g. Valio Ltd. Estonian patent application EE200300351 discloses composition of bacteria (*L. rhamnosus* GG, *L. rhamnosus* DSM LC705 7061, *Propionibacterium freudenreichii* ssp. Shermani DSM7067and *Bifidobacterium infantist* DSM13642) together with galacto-oliogosaccharides (GOS) for improvement of general health status and stimulation of immune system. No data is presented which components of immune system were stimulated, however from the publication it turned out that the best stimulators of TNF-alfa and IL-6 were *Lactobacillus* GG and another *L. rhamnosus* strain E509 (Miettinen M., Vupio-Varkila J, Varkila K. Production of TNF-alpha and interleukin -6 and interleukin -10 is induced by lactic acid bacteria. Infection & Immunity, 1996, 64: 5403-5405).

Polyamine spermidine has inflammation-lowering property. It has been demonstrated that spermidine, when added to human monocytes stimulated with lipopolysaccharides, inhibits effectively the synthesis of TNF, IL-1, IL-6 and other proinflammatory cytokines (Zang M, Caragine T. Wong H et al. Spermine inhibits proinflammatory cytokine synthesis in human mononuclear cells: a counter regulatory mechanism, that restrains the immune response. J. Exp. Med., 1997, 185: 1759-1768). Matsumoto with co-authors described the suppression of proinflammatory cytokine synthesis (Matsumoto M, Ohisshi H, Benno Y Impact of LKM512 yoghurt on improvement of intestinal environment of the elderly. FEMS immunol. Medical Microbiol, 2001; 31:181-186). Excessive suppression of proinflammatory cytokine synthesis could be harmful, as it lowers activation of lymphocytes and natural killers acting against cancer cells. *Bifidobacterium lactis* LKM512 comprising yoghurt administration to elderly decreased the glucoprotein haptoglobuline caused inflammatory acute phase response due to IL-1, II-6 and TNF-alfa, but the probiotic administration was also accompanied by decrease of mutagenicity of the intestinal epithelial cells. Besides, polyamines improve the apoptosis of epithelial cells in order to avoid hyperproliferation, an essential prerequisite to colon cancer development (Hamilton-Miller JMT, Probiotics and prebiotics in the elderly. Postgrad. Med., 2004; 80:447-451; Gill HS, Darragh AJ, Cross M. Optimizing immunity and gut function in the elderly. J. Nutr.Health Aging 2001, 5: 60-91). Due to the aforementioned effects the consumption of polyamines-rich diets by elderly is contradictory. At the same time it is evident, that different lactic acid bacteria incl. lactobacilli species and strains differ by their ability to induce pro-and anti-inflammatory cytokines and non-specific cellular immune response. Up to now, no lactobacillus species/strain has been described, which would be able to produce physiologically relevant amounts of polyamines, which could be detected in urine after the consumption of this particular strain comprising composition and which promote simultaneously the adaptive activation of immunocytes due to interleukin IL-6.

Proinflammatory cytokine IL-6 synthesis has been described after 24h of stimulation with different strains of *Bifidobacterium animalis* and *Lactobacillus rhamnosus* (Miettinen M., Vuopio-Varkila J, Varkila K. Synthesis of human tumour necrosis factor alpha, interleukin-6 and interleukin-10 is induced by lactic acid bacteria. Infection and Immunity, 1996, 64:5403-5408). It is important to observe inflammation markers like counts of leucocytes (WBC) and amount of CRP in sera on the induction of IL-6 (Kiecolt-Glaser JK, Preacher KJ, MacCallum RC et al. Chronic stress and age-related increases in the proinflammatory cytokine IL-6, PNAS, 2003; 100:9090-9095) to avoid the overproduction of IL-6. Aforementioned is associated with cardio-vascular diseases, arthritis, type II diabetes, cancer, periodontal diseases, cachexy and decrease of organisms functions (Rose-John S., J. Scheller, G. Elson, and S. A. Jones. Interleukin-6 biology is coordinated by membrane-bound and soluble receptors: role in inflammation and cancer J. Leukoc. Biol., August 1, 2006; 80 (2): 227 - 236).

*Lactobacillus plantarum* is a widely spread representative of the genus *Lactobacillus.* Aforementioned lactobacillus species is present on fermented plants (sauerkraut, pickles, and silage), fermented dairy/meat products (cheese, salami) as well as in human gastrointestinal tract (Bottazzi, V. Other fermented dairy products. Food and feed production with microorganisms. Biotechnology. Verlag Chemie, Weinhein, Ed. Reed G. 1983, 5: 315-364; Hammes, W. P., Weiss, N., Holzapfel, W. The genera Lactobacillus and Carnobacterium. The Procaryots. Springer - Werlag, Heidelberg, New York, Eds. Balows A., Trüpes H. S., Drowkin M., Schleifer K-H., 1992; 2: 1535-1594; Xanthopoulos, V., Hatzikamari, M., Adamidis, T., Tsakalidou, E., Tzanteakis, N., Litopoulou-Tzanteaki, E. Heterogeneity of Lactobcillus plantrum isolates from Feta cheese througout ripening. J.Appl. Microbiology 2000, 88: 1056-1064). *Lactobacillus plantarum* is able to reorganize its metabolism according to environmental conditions.

Probiotic *Lactobacillus plantarum* is available in probiotic foods as well as in food supplements (e.g. *Lactobacillus plantarum* 299v DSM 9843, Probi AB, Sweden, Skånemejeriers' ProViva probiotic brand in Sweden or as one of the components in bacterial composition VSL#3 (VSL Pharmaceuticals, Inc. USA). WO2007/108764 (Probac AB) discloses the action mechanisms of *Lactobacillus plantarum* stains, which are able to enhance immunotolerance in the case on autoimmune coeliac disease. Korean patent applications KR20020072913 and KR20050080630 discloses a mix of lactobacilli, where belongs also *Lactobacillus plantarum,* at least one of its cytoplasm fraction or the strain itself is able to inhibit cell lines of stomach and colon cancer.

Cheese as a probiotic carrier has several controversial aspects. Incorporation lactobacilli of human origin into a food product different from other milk- based products and having a long ripening period could be complicated (Gardiner, G, Ross, R. P, Collins, J. H, Fitzgerald, G, Stanton. C. Development of a probiotic cheddar cheese comprising human derived Lactobacillus paracasei strains, Appl. Environ. Microbiol., 1998, 64,. 6: 2192-2199; Madkor, S., A., Tong, P.S., EI Soda, M. Ripening of Cheddar cheese with attenutaed adjunct cultures of Lactobacilli. J. Dairy Sci. 1999; 1684-1691). At the same time the fat and protein-rich cheese matrix protects a probiotic microbial strain throughout the passage of gastrointestinal tract better than other milk products (yoghurt, kefir). Antimicrobial and antioxidative probiotic cheese has been produced by using *Lactobacillus fermentum* ME-3 (DSM 14241) (Estonian patent EE04580, Russian patent RU2284354, USA patent US6190879). USA patent US6190879 discloses microorganism from the genus *Streptomyces* for production of transglutaminase and a method for incorporation putrescine into casein during cheese production.

European patent EP1064857B1 (Snow Brand Milk products Co Ltd., 2004) discloses methods for production substances incl. putrescine with lactobacilli, bifidobacteria and propionibacteria from Gouda cheese milk ultrafiltration. These methods are either polymerization reactions for incorporating putrescine into casein or *vice versa -* purification of these compounds by ultrafiltration, that have already been produced into milk, methods are different from this one described in present invention, where the putrescine, that has been produced by lactobacilli into cheese milk is still present in cheese after 30 days of ripening. Various non-starter lactobacilli have been described (*Lactobacillus paracasei, Lactobacillus curvatus*), which are able to gain energy for proliferation from ornithine (ornithine is released from milk casein arginine) after depletion of carbohydrates (Laht T.-M., Kask S., Elias P., Adamberg K., Paalme T. Role of arginine in the development of secondary microflora in Swiss-type cheese. Int. Dairy Journal, 2002, 12: 831-840).

Thus, till now no lactobacillus species/strain have been described, the culture of which produces NO and additionally physiologically relevant amounts of polyamines in food product, whereas the latter could be detectable in urine after the consumption of this strain comprising food product (cheese) or composition and that is able to regulate through polyamines the apoptosis of intestinal epithelium and increase the count of the mucosal lymphatic follicles and blood monocytes, regulating the condition of mucosa by NO and antioxidative compounds and to enhance the activation of immune cells particularly due to the activation of macrophages by central interleukine.

### DISCLOSURE OF THE INVENTION

The present invention relates to a novel isolated microorganism strain *Lactobacillus plantarum* Inducia DSM 21379 as probiotic, its functional properties, food product (e.g. cheese) and composition comprising the said microorganism and application of the said microorganism for production of medicine for enhancing cellular immunity. *L. plantarum* Inducia DSM 21379 produces polyamines from ornithine and glutamate, nitric mono-oxide (NO) from arginine, possesses antioxidative activity and with produced polyamines, nitric mono-oxide and antioxidative compounds improves the intestinal barrier function, increases the number of immunocytes in blood and induces cytokine synthesis for enhancement of organisms' natural defence.

The *Lactobacillus plantarum* strain DSM 21379, which is the object of the investigation, was isolated from a faecal sample of a healthy child during a comparative study of the microflora of Estonian and Swedish children. The microorganism strain *Lactobacillus plantarum* Inducia DSM 21379 was isolated by seeding the dilutions of the faeces of a healthy one-year old Estonian child (10⁻²-10⁻⁷ in phosphate buffer with 0.04% thioglycol acid; pH 7.2). The dilutions were seeded on freshly prepared MRS agar medium and cultivated at 37°C microaerobically. The strain, which is the object of the investigation, was isolated according to the characteristic morphology of colonies and cell to *Lactobacillus sp.* A provisional and more precise identification followed as described next.

The fact that the microbial strain originates from the intestinal tract of a healthy child proves its GRAS (generally recognized as safe) status i.e. that this strain of microorganism is harmless for human organism and is suitable for oral application.

**Cultural - morphological characteristics** were detected after cultivation on MRS agar and in MRS broth (OXOID). Cells of *Lactobacillus plantarum* Inducia DSM 21379 are Gram-positive, non-spore-forming rods of regular shape, occurring singly, in pairs or in short chains.

### Physiological-biochemical characteristics

The MRS broth was suitable for cultivation of the strain during 24- 48 h in microaerobic environment, after which homogenous turbid growth occurred in the broth. Colonies on MRS agar plates after 48h of growth at 37°C in microaerobic conditions (atmosphere CO₂/O₂/N₂: 10/5/85) are round, 2-2.5 mm of diameter, sooth, entire, convex and white.

The optimal growth temperature is 37°C; it multiplies also at 15°C and 45°C. The optimal pH of the growth environment is 6.5.

*Lactobacillus plantarum* strain Inducia DSM 21379 is catalase and oxidase negative, facultatively heterofermentative, no gas is produced from glucose and no arginine hydrolysis.

Strain *Lactobacillus plantarum* Inducia DSM 21379 was identified on the basis of biochemical activity with API 50CHL System (bioMérieux, France) kit as *Lactobacillus plantarum* (Coincidence with the type strain: *excellent,* ID %-99.9, T index -0.81) and molecular identification by ITS-PCR (*Internal-Transcribed Spacer Polymerase Chain Reaction*). The comparison with the reference strain *Lactobacillus plantarum* ATCC 14917 confirmed the preliminary identification by API 50CHL

Carbohydrate utilization profile of *Lactobacillus plantarum* Inducia DSM 21379 according to API CHL 50 is following. Positive reaction for: ribose, galactose, D-glycose, D-fructose, D-mannose, mannitol, sorbitol, α methyl-D-mannoside, a methyl-D-glucoside, N acetyl-glucosamine, amygdalin, arbutine, esculine, salitsin, cellobiose, maltose, lactose, melibiose, sachharose, trehalose, melezitose, β-gentiobiose, D-turanose, gluconate. Weak reaction for starch.

Negative reaction for L- arabinose, D-raffinose glycerol, erythrol, D-arabinose, D-xylose, L-xylose, adonitol, β methyl-xyloside, L-sorbose, rhamnose, dulcitol, inositol, inulin, glycogen, xylitol, D-lyxsose, D-tagatose, D-fucose, L-fucose, D-arabitol, L-arabitol, 2 keto-gluconate, 5 keto-gluconate.

According to API ZYM test-kit (bioMérieux, France) *Lactobacillus plantarum* Inducia DSM 21379 possesses leucine arylamidase, valine arylamidase, acid phosphatase, naphthol-AS-BI-phosphohydrolase, α-glucosidase, β -glucosidase activities and acetoin activity. Weak reaction for valine arylamidase, naphthol-AS-BI-phosphohydrolase, β-galactosidase, cystine arylamidase, esterase (C4), esterase (C8), N-acetyl- β-glucosaminidase was detected.

The molecular identification of *Lactobacillus plantarum* Tensia DSM 21379 was confirmed by Internal-Transcribed Spacer Polymerase Chain Reaction (ITS-PCR). The banding pattern of the isolates was visually compared with that of the reference strain *Lactobacillus plantarum* ATCC 14917 (Fig. 1).

Method. Strain identification was confirmed by (ITS-PCR) Internal-Transcribed Spacer Polymerase Chain Reaction in comparison with the reference strain *Lactobacillus plantarum* ATCC 14917.

The DNA extraction from *Lactobacillus* isolates was performed using lysozyme (Serva, Sweden; 20 mg/ml), mutanolysin (Sigma; 0.5 mg/ml) and proteinase K solutions (Fermentas, Lithuania; 14.6 mg/ml).

The DNA amplification was performed in a reaction volume of 50 µl containing 1x*Taq* polymerase buffer (Fermentas, Lithuania), 1.5U *Taq* polymerase (Fermentas), 0.5 µM of each primer (16S-1500F and 23S-32R; DNA Technology AS), 200 µM deoxynucleoside triphosphates (Amersham Pharmacia Biotech, Germany), 2 mM MgCl₂ and 2 µl of extracted DNA. Subsequently, the PCR product was restricted using a *Taq* I restriction enzyme (Fermentas Lithuania). DNA fragments were separated by electrophoresis (1.5 h, 100 V) on a 2% agarose gel in 1xTBE [Tris(Hydroxymethyl)aminomethane-borate/disodium ethylendiamine tetraacetate] buffer. The banding patterns were visualized and visually compared with reference stain *L. plantarum* ATCC 14917.

*Lactobacillus plantarum* Inducia was deposited in Deutsche Sammlung von Mikroorganismen und Zellkulturen GmbH under the registration number DSM 21379 at 16.04.2008.

### Pulsfield-gel-electrophoresis (PFGE) profile

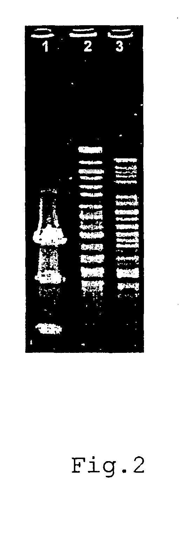

**Method**. For pulsfield-gel-electrophoresis (PFGE) procedure the lactobacillus strains were grown in MRS broth at 37°C for 24 h. The cells were washed in SE buffer (75mM NaCl, 25mM EDTA, pH=7.4), density of the cell suspension was adjusted to 1.5 (OD₆₀₀). The DNA extraction from *Lactobacillus* isolates was performed in EC buffer (50mM EDTA (pH 8.5), 0.5% Na-laurylsarcosine, 0.2% Na-deocycholate 2mg/ml lysozyme, 10U mutanolysin), followed by 1mg/ml proteinase K solution (100 mM EDTA-1% sarcosyl-0.2% deoxycholate, pH 8.0) containing buffer. Lysed probes were washed in TE buffer and cut to 2 mm, extracted overnight with enzyme 50U Not I (Bio-Rad). The electrophoresis was carried out in CHEF-DR II (Bio-Rad) for 22h at 14°C. The banding patterns were visualized in UV light illuminator (Fig.2).

### Antibiotic resistance

**Method.** *Lactobacillus plantarum* Inducia DSM 21379 was tested using antibiotic strips of the E-test (AB Biodisk, Solna). The MIC (minimal inhibitory concentration) was determined according to European Commission (EUC) suggested epidemiological break-points.

**Table 1. Antibiotic resistance of Lactobacillus plantarum Inducia DSM 21379**

| **Antibiotic** | MIC* (µg/ml) | MIC* (µg/ml) | EUC antibiotic resistance MIC* breakpoint values (µg/ml) |
|---|---|---|---|
| | *L. plantarum* Inducia DSM 21379 | Control strain *L. plantarum* DSM 21380 | |
| Ampicillin | 0.19 | 0.25 | 4 |
| Gentamicin | 1 | 1.5 | 64 |
| Streptomycin | 6 | 16 | 64 |
| Erytromycin | 0.25 | 0.19 | 4 |
| Clindamycin | 0.016 | 0.032 | 2 |
| Tetracycline | 6 | 8 | 32 |
| Chloramphenicol | 2 | 2 | 8 |
| Cipro/ofloxacin | 32 | 32 | 4 |

| | | | |
|---|---|---|---|
| * minimal inhibitory concentration | | | |

*In vitro* study of antibiotic susceptibility showed that *Lactobacillus plantarum* Inducia DSM 21379 did not express antibiotic resistance against most important antimicrobial preparations. A somewhat higher MIC for ciprofloxacin was detected, revealed in normal range of wild strains described previously (Vankerckhoven V, Huys G, Vancanneyt M, Vael C, Klare I, Romond M-B,. Entenza J M, Moreillon P, D. Wind R, Knol J, Wiertz E, Pot B., Vaughan E. E, Kahlmeter G, Goossens H. Biosafety assessment of probiotics used for human consumption: recommendations from the EU-PROSAFE project. Trends in Food Science & Technology 2008; 19: 102e114). Therefore, no horizontal transfer of antibiotic resistance genes of *Lactobacillus plantarum* Inducia DSM 21379 during the application of the strain as a probiotic could be predicted.

### Antimicrobial activity

*Lactobacillus plantarum* Inducia DSM 21379 expresses *in vitro* on MRS agar medium antagonistic activity against several enteric pathogens (Table 2).

**Table 2. Lactobacillus plantarum Inducia DSM 21379 antimicrobial activity against pathogens and non-starter lactobacilli on modified MRS agar medium (pathogen growth inhibition zone, mm)**

| **Pathogen** | **Growth inhibition zone (mm)** |
|---|---|
| Non-starter lactobacilli (NSLAB) | 2.67±3.4 |
| *Listeria monocytogenes* | 22.7 ± 2.4 |
| *Yersinia enterocolitica* | 11.2±2.7 |
| *Salmonella enteritidis* | 22.1 ± 1.9 |
| *S*. *typhimurium* | 20.8 ± 2.8 |
| *Shigella sonnei* | 24.0 ± 0.1 |
| *Escherichia coli* | 23.0 ± 1.4 |
| *Enterobacter sakazakii* | 18.1 ± 1.8 |
| *Campylobacter jejuni* | 12.0 ± 7.6 |

*Lactobacillus plantarum* Inducia DSM 21379 antimicrobial activity *in vitro* in streak-line procedure (antimicrobial effect of metabolites) was highest against *E. coli,* followed by growth inhibition of *Salmonella* sp., *Shigella* and *Listeria.* The lowest antimicrobial activity was detected against other lactobacilli (NSLAB).

### Functional properties

### The profile of metabolites

**Method**. The profile of *Lactobacillus plantarum* Inducia DSM 21379 metabolites was determined by gas chromatographic method (Hewlett-Packard 6890) after incubation in microaerobic milieu for 24 h and 48h (Table 2). *L. plantarum* strain was grown on MRS agar for 48h microaerobically (10% CO₂). A suspension (McFarland 4.0 turbidity standard, 10⁹CFU/ml) of lactobacillus culture was prepared in 0.9% NaCl solution. 1.0 ml of this suspension was transferred to 9.0 ml of MRS broth. The metabolite concentration (mmol/l) was detected using the capillary column HP-INNOWax (15 m ×0.25 mm; 0.15 µm). The column temperature program was 60°C 1 min, 20°C/min 120°C 10 min; detector (FID) 350°C.

Electrochemical measurements of H₂O₂ were carried out with 24h old intact cells in 500µl of MRS broth with Apollo 4000 free radical analyzer (WPI, Berlin, Germany) and electrodes of type ISO-HPO2 and ISO-NOP.

ISO-HPO2 electrode signals were registered during 5-7 minutes. Mean signal strength was calculated. Each experimental point was measured in 4 independent parallels and each parallel was measured twice. H₂O₂ concentration was calculated according to the standard curves correlation with the strength of the electrodes signal.

**Table 3. Acetic acid, lactic acid and succinic acid concentration (mmol/l) of Lactobacillus plantarum DSM 21379 Inducia in MRS broth at microaerobic cultivation for 24 and 48h and H₂O₂ concentration (µg/ml) of intact cells**

| | Acetic acid (mmol/l) | | Lactic acid (mmol/l) | | Succinic acid (mmol/l) | | H₂O₂ (µg/ml) |
|---|---|---|---|---|---|---|---|
| | 24 h | 48 h | 24 h | 48 h | 24 h | 48 h | intact cells 24 h |
| *L.plantarum* Inducia DSM 21379 | 2.1 | 2.4 | 133.3 | 186.6 | 0.6 | 0.6 | 288.9 ± 175.8 |
| Control strain *L.plantarum* DSM 21380 | 1.4 | 1.7 | 112.2 | 129.2 | 0.6 | 0.6 | 196.4 ± 128.8 |

*Lactobacillus plantarum* Inducia DSM 21379 produces substantial amounts, of acetic acid, lactic acid and H₂O₂ in comparison with the control and which are essential for the trophic of the colon mucosa.

### Total antioxidative activity of Lactobacillus plantarum Inducia DSM 21379

**Method**. For the detection of TAA and TAS of the microbial cells, the strain *L*. *plantarum* Tensia was incubated in MRS broth (Oxoid, U.K.) for 24h at 37°C. Microbial cells were harvested by centrifugation (1500 RPM, during 10 min) at 4°C and the pellet was washed with isotonic saline (4°C) and suspended in 1.15% KCl (Sigma, USA). The density of the suspension was OD ₆₀₀ of 1.1 x 10⁹ bacterial cells ml⁻¹). Total antioxidative activity (TAA) was assessed by using the linolenic acid test (LA-test). (Kullisaar, T, Songisepp, Mikelsaar M, Zilmer, K, Vihalemm, T, Zilmer, M.. Antioxidant probiotic fermented milk decreases oxidative stress-mediated atherogenicity in human. British J of Nutrition 2003, 90, 2, 449-456) and total antioxidative status (TAS) was measured by commercial kit (TAS, Randox Laboratories Ltd.; UK).

**Table 4. Total antioxidative activity (TAA) and total antioxidative status (TAS) of Lactobacillus plantarum Inducia DSM 21379**

| Strain | TAA (%) | TAS (mmol/l) |
|---|---|---|
| *L. plantarum* Inducia DSM 21379 | 26±1.2 | 0.13±0.04 |
| *L. plantarum* DSM 21380 | 22±5 | 0.05±0.02 |

### Production of nitrogen mono-oxide (NO)

**Method**. Nitrogen mono-oxide production measurements were carried out with 24h old intact cells in 500 µ of MRS broth with Apollo 4000 free radical analyzer and electrodes (WPI, Berlin, Germany). ISO-NOP electrode signals were registered during 5-7 minutes. Mean signal strength was calculated. Each experimental point was measured in 4 independent parallels and each parallel was measured twice. NO concentration was calculated according to the standard curves correlation with the strength of the electrodes signal.

**Table 5. NO concentration (µM) produced by Lactobacillus plantarum DSM 21379**

| **Strain number** | **NO concentrations (µM)** |
|---|---|
| *L. plantarum* Inducia DSM 21379 | 2.7 ± 1.2 |
| *L. plantarum* DSM 21380 | 2.6 ± 0.8 |
| *L. coprophilus* | 2.1 ± 1.1 |
| *L. plantarum* | 2.1 ± 0.9 |
| *L. paracasei ssp paracasei* strain no 1 | 1.3 ± 0.8 |
| *L. paracasei ssp paracasei* strain no 2 | 1.8 ± 0.9 |
| *L. paracasei ssp paracasei* strain no 3 | 2.8 ± 1.6 |
| *L. buchneri* | 2.0 ± 1.1 |

*Lactobacillus plantarum* Inducia DSM 21379 was the best NO producer among the tested heterofermentative lactobacilli.

### In vitro polyamines production of Lactobacillus plantarum Inducia DSM 21379

**Method**. Microbial strains were suspended in physiological saline according to McFarlandi turbidity standard (10⁹ CFU/ml) and 0.5 ml of each strain suspension was seeded into decarboxylation medium (á 4.5 ml) and incubated at 37°C for 4 days (Bover-Cid and Holzapfel W.H. Improved screening procedure for biogenic amine production by lactic acid bacteria. Int J food Microbiol 1999; 53, (1); 33-41(9)).

For detection of BA 200 µl of medium was derivatized for GC analyze by modified method of Nakovich (Nakovich, L. Analysis of biogenic amines by GC/FID and GC/MS. Thesis, Virginia polytechnic institute, USA. 2003).

GC analysis were carried out by gas chromatograph HP 6890 Series GC System, with capillary colonna HP-5 19091J-413 (30 m ×0.32 mm; 0.25 µm) with 160°C 1 min, 20°C/min 280°C 15 min; and detector (FID) 300°C.

**Table 6. Production of polyamines in vitro in the decarboxylation medium (Arena, M.E. and Manca de Nadra, M. C. Biogenic amine production by Lactobacillus. J Appl Microbiol, 2001; 90; 158-162)**

| Sample | Polyamines (µg/ml) and biogenic amines | | | | | | | | |
|---|---|---|---|---|---|---|---|---|---|
| | Arginine | | Glutamine | | Lysine | | Ornithine | | Histidine |
| | Putrescine | Cadaverine | Putrescine | Cadaverine | Putrescine | Cadaverine | Putrescine | Cadaverine | Cadaverine |
| *L. plantarum* Inducia DSM 21379 | 0 | 0.4 | 1.2 | 0.5 | 0 | 0.4 | 1,9 | 0 | 0 |
| *L. plantarum* DSM 2137980 | 0 | 0 | 0 | 0 | 0 | 0.3 | 0.5 | 0.6 | 0 |

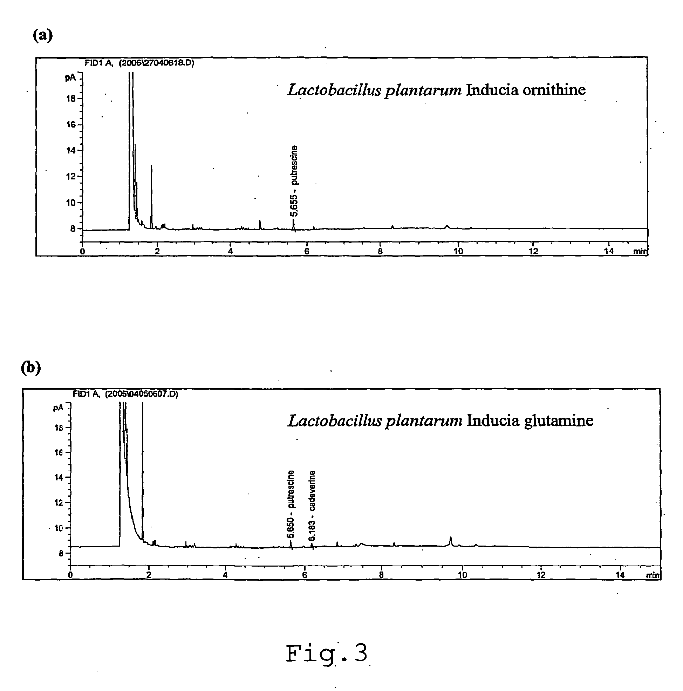

*Lactobacillus plantarum* strain Inducia DSM 21379 was able to produce cadaverine from arginine, putrescine both from glutamine as well as from ornithine. But the control strain was able to produce putrescine in low amounts only from ornithine (Fig.3). Traces of cadaverine were detected both in control strain as well as in *Lactobacillus plantarum* Inducia DSM 21379. No histamine production was detected (Table 6).

### DESCRIPTION OF THE DRAWINGS

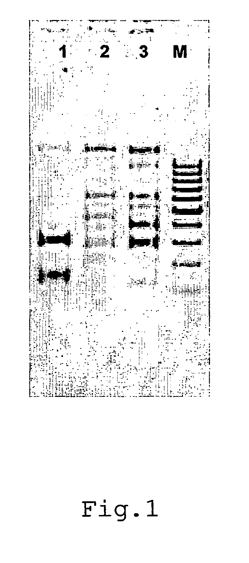

Fig.1. Molecular identification of the strain by ITS-PCR
   *1. L.plantarum* Inducia DSM 21379
   *2. L. plantarum* DSM 21380
   *3. L. plantarum* CRL 972 (ATCC 14917)
   M- 100bp marker (Fermentas)
Fig. 2. *L. plantarum* Inducia DSM 21379 molecular fingerprints in comparison with *L*. *plantarum* control strain (Pulse field-gel-electrophoresis profile, PFGE).
   1. Lambda Ladder PFG Marker (New England Bio Labs *Inc.*)
   *2. L. plantarum* DSM 21380 (control)
   3. *L. plantarum* DSM 21379
Fig.3. Production of polyamines by *L. plantarum* DSM 21379 *in vitro* in the decarboxylation medium (Arena, M.E. and Manca de Nadra, M. C. Biogenic amine production by *Lactobacillus.* J Appl Microbiol, 2001; 90; 158-162) (a) from ornithine (b) from glutamine
Fig. 4. Production of polyamines in cheese by *Lactobacillus plantarum* DSM 21379 (detected according to the method of Nakovich, L. Analysis of biogenic amines by GC/FID and GC/MS. Thesis, Virginia Polytechnic Institute, USA. 2003)
Fig. 5. Lactobacillus species by Pearson UPMAG clusteranalyses in *L. plantarum* Inducia DSM 21379 group.
   Subjects no 3, 6, 9, 11, 14, 15, 19 and 20.
   Four samples: at the recruitment (1), after consumption of probiotic cheese (2), after *wash-out* period (3) and after consumption of control cheese (4).
Fig.6. IL-6 and blood monocytes
   Positive correlation between IL-6 and blood monocytes in last (IV) sample - r=0.653, p=0.029, n=11. The linear regression model confirmed the correlation (R² =0.405, R² adj = 0.338, = 0.035).

### DESCRIPTION OF THE EMBODIMENTS

### Example 1. Obtaining optimal viable counts of Lactobacillus plantarum Inducia DSM 21379 in food product

### Test with Estonia cheese

**Method**. Microorganism *Lactobacillus plantarum* Inducia DSM 21379 was added to the cheese milk of Dairy Cooperative E-Piim, (inoculation dose 3x10⁸ CFU/vat) and the milk was renneted (25 min). The curds were cut (25 min), heated (34°C 15 min), dried (25 min), pressed, drained (1 h), salted in brine (12°C; 20% NaCl; pH 4,7) 20 h, drained and dried (8 h), backed into plastic and ripened at 12°C for at least 4 weeks.

**Table 7. Dynamics of microflora in Lactobacillus plantarum Inducia DSM 21379 comprising cheese.**

| Sample | *L. plantarum* Inducia DSM 21379 comprising cheese | | | Control cheese | | |
|---|---|---|---|---|---|---|
| | Day 14 | Day 21 | Day 30 | Day 14 | Day 21 | Day 30 |
| total counts | 8x10⁸ | 4x10⁸ | 10¹⁰ | 2x10⁸ | 3x10⁸ | 2x10⁸ |
| *Lactobacillus SP* | 5x10⁸ | 2x10⁸ | 10¹⁰ | 10⁵ | - | 3x10³ |
| *L. plantarum* | 5x10⁸ | 2x10⁸ | 10¹⁰ | - | - | 3x10³ |
| cocci | 3x10⁸ | 2x10⁸ | 10⁹ | 2x10⁸ | 3x10⁸ | 10⁸ |
| spore-forming microbes | - | - | 10³ | - | 10⁷ | 10⁸ |
| *L. casei* | - | - | - | 10⁵ | . | - |

### Example 2. Production of polyamines in food product by Lactobacillus plantarum Inducia DSM 21379

**Method**. Cheese samples were extracted (20 ml 50% methanol solution was added to 10g of cheese and incubated at 45°C for 1 h, cooled to 30°C and centrifuged) and 200µl of upper layer was derivatized for GC analyze by modified method of Nakovich (Nakovich, L. 2003 Analysis of biogenic amines by GC/FID and GC/MS) in Department of Microbiology of the University of Tartu.

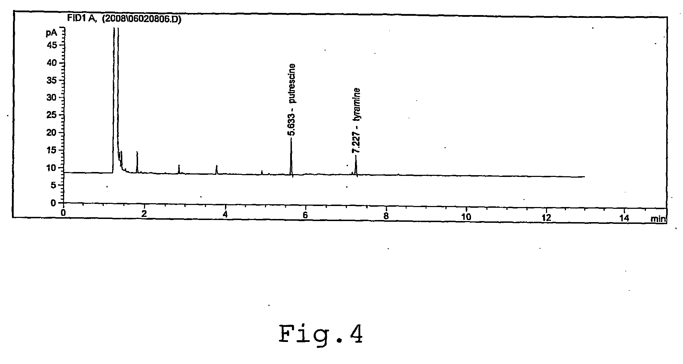

GC analysis were carried out by gas chromatograph HP 6890 Series GC System, with capillary colonna HP-5 19091J-413 (30 m ×0,32 mm; 0,25 µm). The column temperature program 160°C 1 min, 20°C/min 280°C 15 min; and detector (FID) 350°C (Fig. 4).

The production of putrescine and tyramine was related to that: in different lots in comparison with control-cheese putrescine content increased 3-11 times and tyramine content accordingly 2-5 times.

*Lactobacillus plantarum* Inducia DSM 21379 belongs to the facultatively heterofermentative group of lactobacilli and therefore the content of tyramine in cheeses was significantly lower than that of strains of OHEL group. On the other hand, the content of putrescine was higher.

**Table 8. Biogenic amines and polyamines in Lactobacillus plantarum Inducia DSM 21379 comprising test-cheeses from industrial test-trials**

| Sample | Viable counts of strain incorporated into cheese (CFLT/g) at day 3-4 after preparation. | Amines (mg/kg) | | | Viable count of *L*. *plantarum* Tensia DSM 21379 in rine |
|---|---|---|---|---|---|
| | | Tyramine | Putrescine | Cadaverine | |
| *L. plantarum* Inducia DSM 21379, 1. Batch | 3x10⁸ | 4.83 | 20.28 | 0 | 2x10 |
| Control cheese 1. Batch | - | 2.31 | 1.82 | 0 | - |
| *L. plantarum* Inducia DSM 21379, 2. Batch | 3,3x10⁷ | 13.57 | 24.67 | 0 | 2x10⁶ |
| Control cheese 2. Batch | - | 2.63 | 6.64 | 0 | - |

Permitted concentration of tyramine in food e.g. in cheese is 200 mg/kg (Karovicova and Kohajdova. Biogenic amines in food. Chem pap. 2005; 59 (1); 70-79; Larqué, M., Sabater-Molina, S. Zamora E. Biological significance of dietary polyamines. Nutrition 2007; 23(1): 87-95). Tyramine is considered toxic in concentrations of 1000-8000 mg/kg. Putrescine is considered toxic if detected in organism in concentration of 2000 mg/kg per body weight and total toxicity of polyamines is > 300 mg/kg per food product (Larqué, M., Sabater-Molina, S. Zamora E. Biological significance of dietary polyamines. Nutrition 2007; 23(1): 87-95).

By consumption of 100 g *Lactobacillus plantarum* Inducia DSM 21379 comprising cheese the subject gets ca 3 mg of putrescine. Thus, in the case of person of 70 kg the concentration is up to 50 µg per kg, which does not express toxic effect.

### Example 3. Enhancement of defence capability of intestinal mucosa with Lactobacillus plantarum Inducia DSM 21379 comprising food

In the experimental model with NIH mice 3 groups of mice consumed different cheeses during 30 days (control cheese with no additives, *Lactobacillus plantarum* strain Inducia DSM 21379 2x10⁸ cfu /g comprising cheese).

Cheese was administered to mice at night (normal awake time for mice) 4.4g/per mouse, daily *ad libitum* regular commercial diet. Consumed amount of cheese was 3.5-4.2 g/per night.

Mice stayed in good condition, no changes in skin, fur and digestion was detected. Cheese administration caused increase of body weight: body weight at the beginning of the trial was 22.9-29.8g and at the end of the trial a weight gain was up to 2-6.1 g. The mice were sacrificed by cervical dislocation at day 30. No translocation of lactobacilli or other bacteria of the microbiota to blood or organs was detected at the autopsy.

**Table 9. Total counts of lactobatsilli in faeces, ileum and colon**

| Sample | Control group (cheese without *L*. *plantarum* Inducia DSM 21379 was administered) | Test group (cheese comprising *L. plantarum* Inducia DSM 21379 was administered) |
|---|---|---|
| Faecal samples | | |
| Day 0 | 6.7 | 7.6 |
| Day 10 | 8.0 | 8.3 |
| Day 15 | 7.0 | 8.0 |
| ileum | 3.0-7.1 / 5.95* | 6.3-7.7 / 6.95* |
| colon | 4.4-7.3 / 6.65* | 6.9-7,8 / 7.45* |

| | | |
|---|---|---|
| * Student t-test p= 0,001 | | |

With seeding from ileum and colon from the mice administered with *Lactobacillus plantarum* Inducia DSM 21379 total counts of lactobacilli were found to be increased significantly both in ileum and colon.

Tissue samples from the liver, spleen, ileum and colon were fixed in formalin and embedded in paraffin. Microtome-cut tissue samples were stained with hematoxyline-eosine.

No morphological shifts were found in organs (liver and spleen), which proves the safety of *Lactobacillus plantarum* Inducia DSM 21379.

In the ileum the status of intestinal mucosa, count of goblet and Paneth cells and also the formation of follicles and diffuse multiple deposits by lymphocytes was evaluated.

In the colon, the attention was paid to the numerously goblet cells containing surface epithelium and to the characteristics of lymphatic tissue in connective tissue of mucosa.

**Table 10. Patomorphological evaluation of mice organs after administration of Lactobacillus plantarum Inducia DSM 21379 comprising cheese.**

| Mice group | Liver | Spleen | Ileum | Colon |
|---|---|---|---|---|
| *L. plantarum* Inducia DSM 21379 comprising cheese consumed animals | Hyperaemia 6/10* | ii | Lymph follicles 6/10* | Lymph follicles 8/10* |
| Control cheese consumed animals | Hyperaemia 3/10* | Hyperaemia 1/10 | Lymph follicles 3/10* | Lymph follicles 4/10* |

| | | | | |
|---|---|---|---|---|
| * p<0.05 | | | | |

Administration of *Lactobacillus plantarum* Inducia DSM 21379 comprising cheese during 1 month enhanced liver hyperemia and raised significantly lymphatic follicles (immunocytes) of mice ileum and colon in comparison with control mice. These results refer to enhancement of the defence capability of intestinal mucosa and liver functions.

### Example 4. Examination of clinical blood indices of healthy volunteers consumed Lactobacillus plantarum DSM 21379 comprising food and the increase of Lactobacillus plantarum percentage in the intestinal microbiota

The clinical trial with healthy volunteers evaluated the impact of putrescine, NO and antioxidative compounds producing *Lactobacillus plantarum* Inducia DSM 21379 comprising food consumption on (Estonian cheese) 1) safety for the consumer; 2) humoral and cell immunity parameters of blood; 3) effect on intestinal microbiota; 4) and urine metabolites, to detect possible health-promoting effects.

The study group consisted of 12 healthy volunteers, both male and female (M/F 4/ 8) aged 20-48 years. For exclusion criteria diabetes, glucose and glycohemoglobin HbA1c from blood sera were detected. The trial was randomized double-blind cross-over study. Trial started with 3-week consumption of test cheese. Volunteers consumed the test cheese for 3 weeks. After a 2-week washout period, volunteers were crossed over to another 3 weeks of control cheese administration.

*Lactobacillus plantarum* Inducia DSM 21379 content in 30 old test cheeses was 6x10⁷ CFU/g.

Before consumption the test cheese was incubated with *Lactobacillus plantarum* Inducia DSM 21379 for 30 days at 12°C. Regular Estonian cheese of Edam type without *Lactobacillus plantarum* Inducia DSM 21379 served as a control.

The trial was a randomized blinded cross-over placebo controlled trial. Trial started with 3-week consumption of test cheese, followed by 2 week washout period, after which the control cheese was consumed for 3 weeks in a dose 50 g/day.

### Results

### 1) Safety

No discomfort, abdominal pain or other negative symptoms were reported by trial participants. After cheese trial with volunteers, the values of systemic inflammation markers (U-CRP, ultrasensitive CRP, and leucocytes) were not changed and were within the normal range.

The consumption of probiotic *Lactobacillus plantarum* Inducia DSM 21379 cheese did not cause changes in WBC counts (leucogram) (Table 11). No change was also detected in the values of essential allergy marker IgE in comparison with pre-trial period. Consumption of *Lactobacillus plantarum* Inducia DSM 21379 comprising cheese had no negative impact on organisms' kidney and liver function nor affected according parameters (ASAT, ALAT albumine, blood sera creatinine).

Thus in healthy subjects the consumption of *Lactobacillus plantarum* DSM 21379 comprising cheese does not cause systemic inflammation, allergic sensibilisation or causes harm to essential organs.

### Intestinal microbiota

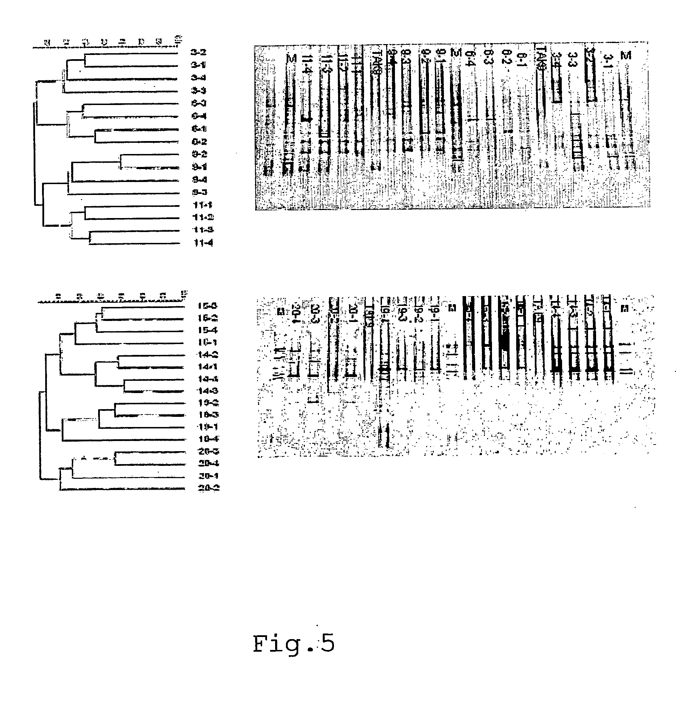

**Method**. Microbial DNA was isolated from cheese by QIAamp DNA Mini Kit (QIAGEN) and amplified with primers Uni-515-GC-rev (ATCGTATTACCGCGGCTGCTGGCA-GC), Lab-159-f (GGAAACAGA/GTGCTAATACCG) (Heilig HG, Zoetendal EG, Vaughan EE, Marteau P, Akkermans, ADL, de Vos WM, /et al./ Molecular diversity of /Lactobacillus/ ssp. and other lactic acid bacteria in the human intestine as determined by specific amplification of 16S ribosomal DNA. /Appl Envir Microbiol /2002; 68: 114-123). Subsequently, the PCR product was separated by DGGE electrophoresis in 30-60% acrylamide containing gel with Dcode^{™} System technique. (Bio-Rad, Hercules, California). Gels were analyzed by BioNumerics 2.5 (Applied Maths, Belgium) software according to Peasoni correlation (Fromin, N.; Hamelin, J.; Tarnawski, S.; Roesti, D.; Jourdain-Miserez, K.; Forestier, N.; Teyssier-Cuvelle, S.; Gillet, F.; Aragno, M.; Rossi, P. Statistical analysis of denaturing gel electrophoresis (DGE) fingerprinting patterns Environmental Microbiology 2002; 4 (11), 634-643) (Fig.5).

### Results

Consumption of *Lactobacillus plantarum* Inducia DSM 21379 comprising cheese changed lactobacilli pattern profile in feces in 5 persons of 12. These changes remained stabile in 3 persons of 5 even 2 weeks after completing the trial.

Thus, consumption of *Lactobacillus plantarum* Inducia DSM 21379 comprising cheese affects the composition of human intestinal lactoflora.

### 3) Humoral and cellular parameters of blood sera

It appeared that *Lactobacillus plantarum* Inducia DSM 21379 comprising cheese induces the raise of cytokine IL-6, in volunteers, which indicates the stimulation of cellular immunity. This finding was confirmed by the increase of blood monocytes at the end of the trial (p=0.015), whereas indices of blood cells were within the normal range.

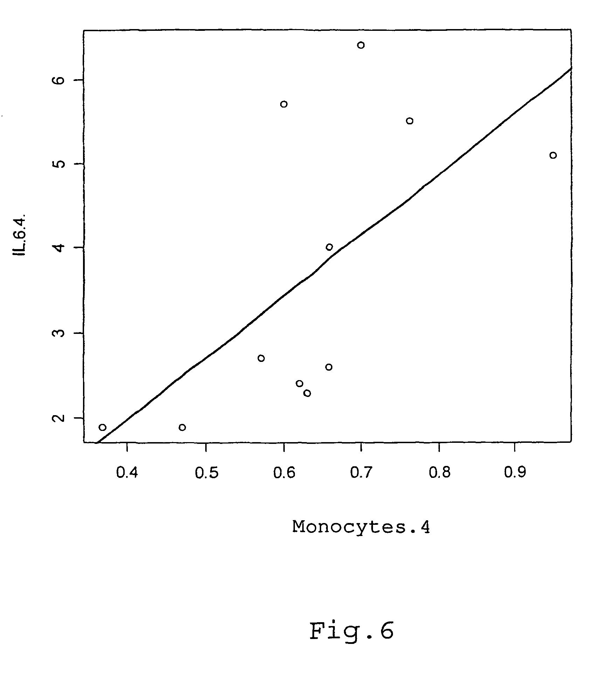

Positive correlation after completing the cheese consumption appeared between IL-6 and blood monocytes (r=0.653, p=0.029, n=11). The linear regression model confirmed the correlation (R² =0.405, R² adj = 0.338, = 0.035) (Fig. 6).

Enhancement of parameters of cellular immunity is in accordance with results on animal model described above; where the administration of *Lactobacillus plantarum* Inducia DSM 21379 induced significantly Peyer's patches i.e. lymph follicles in the intestine. In these follicles interaction of the components of the immune system occurs. Proinflammatory cytokines incl. induction of IL-6 play important role in activation of TH1 type lymphocytes against bacteria through which macrophages i.e. blood monocytes are retroactive activated. Thereupon blood monocytes produce also IL-6.

The increase of the share of cellular immunity is explainable also by the suppression of humoral response e.g. somewhat decreased production of antibodies (IgA, IgG), which however remained within the normal range.

**Table 11. Immunological parameters of blood after consumption of probiotic Lactobacillus plantarum Inducia DSM 21379 comprising cheese**

| Immunity parameters | At the recruitment | End of the trial | *P* values | Normal range and units |
|---|---|---|---|---|
| Ultrasensitive CRP | 1.9 ± 1.6 | 3.3 ± 2.7 | 1.0 | < 5 mg/L |
| Leukocyte counts x10⁹ | 6.3 ± 1.7 | 6.9 ± 1.6 | 0.116 | 4 -10 x 10⁹/L |
| Lymphocytes | 2.3 ± 0.5 | 2.2 ± 0.4 | 0.878 | 0.8 -2.9 x 10⁹/L |
| Monocytes | 0.55 ± 0.17 | 0.64 ± 0.15 | 0.032 | 0.15 -0.75 x 10⁹/L |
| Cytokine IL-6 | 2.7 ± 1.0 | 3.8 ± 1.7 | 0.020 | < 3.4 ng/L |
| IgA antibodies | 2.5 ± 0.9 | 2.3 ± 0.8 | 0.009 | 0.7-4.0 g/L |
| IgM antibodies | 1.3 ± 0.5 | 1.3 ± 0.5 | 0.776 | 0.4-2.3 g/L |
| IgG antibodies | 12.9 ± 3.2 | 12.4 ± 3.0 | 0.017 | 7.0-16 g/L |
| IgE antibodies | 19.6 ± 21.2 | 21.4 ± 25.9 | 0.232 | < 85 kU/L |

### 4) The urine metabolites

For the evaluation of the content biogenic amines before the consumption of the probiotic and the efficiency of the stabilization period, the morning urine and gas chromatography method were used.

Method: urine samples were derivatized with propylchlorophormate for GC analyze by modified method of Uglandi (Ugland HG; Krough M, Rasmussen KE: Aqueous alkylchloroformate derivatization and solid-phase microextraction: determination of amphetamines in urine by capillary gas chromatography. J Chromatography B Biomed Sci Appl 1997; 701:29-38)

GC analysis were carried out by gas chromatograph HP 6890 Series GC System (Hewlett Packard, Avondale, PA, USA), with capillar colonne HP-5 19091J-433 (30 m ×0.25 mm; 0.25 µm) The column temperature program 150°C 1 min, 20°C/min 280°C for 5 min; and detector (FID) 250°C. The biogenic amines concentration was calculated according to nmol / mol creatinine.

**Table 12. Polyamines and biogenic amines content in the morning urine (nmol/mol creatinine) of probiotic cheese consumers**

| | Probiotic cheese comprising *L*. *plantarum* Inducia DSM 21379 | | Control cheese (without *Lactobacillus plantarum* Inducia DSM 21379) | | P values *paired t-test* BL1 vs PRO / BL2 vs PL |
|---|---|---|---|---|---|
| | BL1 mean±stdev range (median) | FRO mean±stdev range (median) | BL2 mean±stdev range (median) | PL mean±stdev range (median) | |
| Put | 0.064 ± 0.072 0 - 0.191 (0.030) | 0.082 ± 0.058 0 - 0.191 (0.077) | 0.043 ± 0.044 0 - 0.126 (0.033) | 0.044 ± 0.060 0 - 0.216 (0.031) | 0.432 / 0.432 |
| acPut | 0.606 ± 0.559 0.151 - 2.104 (0.435) | 1.087 ± 1.451 0.307 - 5.049 (0.447) | 0.796 ± 0.689 0.068 - 2.167 (0.600) | 0.635 ± 0.291 0.154 - 1.219 (0.594) | 0.021 / 0.850 |
| DAP | 0.079 ± 0.092 0 - 0.249 (0.055) | 0.059 ± 0.089 0 - 0.216(0) | 0.056 ± 0.089 0 - 0.253(0) | 0.117 ± 0.142 0 - 0.418 (0.055) | 0.411/0.195 |
| acSpd | 0.251 ± 0.227 0 - 0.813 (0.232) | 0.384 ± 0.198 0.043 - 0.686 (0.384) | 0.354 ± 0.210 0.085 - 0.668 (0.304) | 0.425 ± 0.260 0.065 - 0.831 (0.396) | 0.089 / 0.464 |
| Cad | 0.066 ± 0.123 0 - 0.364(0) | 0.069 ± 0.162 0 - 0.569(0) | 0.067 ± 0.093 0 - 0.293 (0.016) | 0.044 ± 0.085 0 - 0.228(0) | 1.0/0.540 |
| His | 0.231 ± 0.226 0 - 0.595 (0.156) | 0.387 ± 0.524 0 - 1.401 (0.122) | 0.211 ± 0.364 0 - 1.229(0) | 0.478 ± 0.684 0 - 2.093 (0.235) | 0.910 / 0.250 |
| Tyr | 0.153 ± 0.161 0 - 0.476 (0.102) | 0.101 ± 0.132 0 - 0.427 (0.050) | 0.093 ± 0.096 0 - 0.257 (0.093) | 0.212 ± 0.285 0 - 1.035 (0.179) | 0.167/0.149] |

| | | | | | |
|---|---|---|---|---|---|
| Put - putrescine, acPut - N-acetylputrescine, DAP -1.3-diaminopropane, acSpd - N 8-acetylspermidine, Cad - cadaverine; His - histamine, Tyr - tyramine | | | | | |

The content of polyamines (putrescine, acetylputrescine and acetylspermidine) in urine increased after consumption of *L*. *plantarum* Inducia DSM 21379 comprising probiotic cheese. A significant correlation (R= 0.383 p< 0.01, n=48) appeared between the contents of acetylputrescine and acetylspermidine in the urine of volunteers.

At the same time the content of all polyamines incl. putrescine, acetylspermidine and acetylspermine as well as biogenic amines remained within the normal range in the urine.

*Lactobacillus plantarum* strain Inducia DSM 21379 is able to produce putrescine in vitro as well as in cheese. Consumption of probiotic *Lactobacillus plantarum* Inducia DSM 21379 cheese elevated the content of acetylputrescine in urine of trial participants.

Acetylputrescine represents a detoxified compound, elevated content of which proves putrescine production by *Lactobacillus plantarum* Inducia DSM 21379 in gastrointestinal tract of volunteers or absorption and metabolism of additional amounts of putrescine, consumed with cheese. On the other hand, this indicates the successful adaptational reaction of organism to deal with superfluous amounts of putrescine by excreting it with urine in acetylated form.

The immunostimulative effect of putrescine produced by *Lactobacillus plantarum* Inducia DSM 21379 was confirmed by the correlation between blood cytokine IL-6 and the quantity of macrophages (monocytes), which in this case could be considered activated macrophages. The finding mentioned together with H₂O₂ is essential for the organisms' defence against foreign cells (microbes, cancer cells).

Physiological doses of putrescine occurring in the gut due to *Lactobacillus plantarum* Inducia DSM 21379 could theoretically enhance the regeneration of the epithelium of intestinal mucosa and apoptosis of old cells, thus avoiding the hyperproliferation of epithelium. These mechanisms ensure the barrier function of intestinal mucosa and protects against penetration of allergens.

Consumption of *Lactobacillus plantarum* Inducia DSM 21379 cheese regulates the amount and activity of blood monocytes through IL-6 which finding together with lymph follicles (increase of carriers of cellular immunity) demonstrated in experimental animals improves the barrier function of intestinal mucosa and supports organisms' immunological defence functions.

## Claims

1. Isolated microorganism strain Lactobacillus plantarum Inducia DSM 21379 as probiotic that enhances natural defence potential of organism, produces polyamines from ornithine and glutamate, nitric mono-oxide (NO) from arginine, possesses antioxidative activity and improves the intestinal barrier function, increases cellular immunity of the intestinal mucosa and blood and induces adaptively proinflammatory cytokines.

2. The microorganism strain of claim 1 in freeze-dried form.

3. A composition comprising the microorganism according to any of claims 1-2.

4. A food product comprising the microorganism according to any of claims 1-2.

5. The food product according to claim 4, wherein the food product is a dairy product.

6. The food product according to claim 5, wherein the food product is a fermented milk product.

7. The food product according to claim 6, wherein the food product is cheese.

8. Use of microorganism strain according to any of claims 1-2 for production of medicine for enhancing of cellular immunity.

## Patentansprüche

1. Isoliertes *Lactobacillus plantarum* Stamm Inducia DSM 21379 als Probiotikum, das die natürliche Schutzfähigkeit des Organismus erhöht, aus Ornithin und Glutamat Polyamine und aus Arginin Stickstoffmonoxid (NO) bildet, eine antioxydante Wirkung hat und die Schutzfunktion des Gedärms sowie die zelluläre Immunität des Bluts erhöht und adaptiv proinflammatorische Zytokine induziert.

2. Mikroorganismusstamm nach Anspruch 1 in kaltgetrockneter Form.

3. Zusammensetzung welche Mikroorganismen nach einem der vorangehenden Ansprüche 1-2 enthält.

4. Nahrungsprodukt welches Mikroorganismen nach einem der vorangehenden Ansprüche 1-2 enthält.

5. Nahrungsprodukt nach Anspruch 4, wobei dieses Nahrungsprodukt ein Milchprodukt ist.

6. Nahrungsprodukt nach Anspruch 5, wobei dieses Nahrungsprodukt ein fermentiertes Milchprodukt ist.

7. Nahrungsprodukt nach Anspruch 6, wobei dieses Nahrungsprodukt Käse ist.

8. Verwendung des Mikroorganismusstammes nach einem der vorangehenden Ansprüche 1-2 zur Herstellung eines Medikaments, welches die zelluläre Immunität erhöht.

## Revendications

1. La souche isolée de microorganisme *Lactobacillus plantarum* Inducia DSM 21379 comme probiotique qui améliore le potentiel de défense naturelle de l'organisme, produit des polyamines à partir de l'omithine et de glutamate, du mono-oxyde nitrique (NO) à partir de l'arginine, possède une activité antioxydante et améliore la fonction de la barrière intestinale, augmente l'immunité cellulaire de la muqueuse intestinale et du sang et induit de façon adaptative des cytokines pro-inflammatoires.

2. La souche de microorganisme de la revendication 1 dans la forme lyophilisée.

3. La composition comprenant le microorganisme selon l'une des revendications 1 à 2.

4. Le produit alimentaire comprenant le microorganisme selon l'une des revendications 1 à2.

5. Le produit alimentaire selon la revendication 4, dans lequel le produit alimentaire est un produit laitier.

6. Le produit alimentaire selon la revendication 5, dans lequel le produit alimentaire est un produit laitier fermenté.

7. Le produit alimentaire selon la revendication 6, dans lequel le produit alimentaire est un fromage.

8. L'utilisation de la souche de microorganisme selon l'une des revendications 1 à 2 pour la production des médicaments visant à renforcer l'immunité cellulaire.
